# EUROPEAN PATENT SPECIFICATION

(11) **EP 2 389 887 B1**
(45) Date of publication and mention of the grant of the patent: **27.09.2017**
(21) Application number: 11179004.4
(22) Date of filing: 29.07.2008
(51) Int. Cl.: A61B 17/29, A61B 18/14, A61B 90/00

(54) **Polyp removal jaws**
Polypenentfernungsklemme
Mâchoires à suppression de polype

(30) Priority: 30.07.2007 US 881946
(43) Date of publication of application: 30.11.2011
(62) Divisional of application: 08013604.7
(73) Proprietor: Covidien LP, Mansfield, MA 02048 (US)
(72) Inventor: Artale, Ryan, Boulder, CO 80305 (US); Carlton, John, Las Vegas, NV 89118 (US)
(74) Representative: Soames, Candida Jane

(56) References cited:
- EP-A1- 0 543 122
- EP-A1- 0 795 301
- DE-A1- 4 130 064
- DE-U1- 9 215 589
- US-A- 4 671 274
- US-B1- 6 273 887

## Description

### BACKGROUND

### Technical Field

The present disclosure relates to an apparatus for the removal of internal tissue, and more particularly, to jaw members configured for the removal of polyps and the like.

### Background of Related Art

A polyp is an abnormal growth of tissue projecting from a mucous membrane. A polyp that is attached to the surface of the mucous membrane by a narrow elongated stalk is said to be pedunculated. If no stalk is present, the polyp is said to be sessile. Polyps are commonly found in the colon, stomach, nose, urinary bladder and uterus. Polyps may also occur elsewhere in the body where mucous membranes exist like the cervix and small intestine.

The surgical procedure for removing a polyp is generally referred to as a "polypectomy". Polypectomys are generally endoscopic or laparoscopic procedures performed through the oral or anal cavities. When the location of the polyp permits, the polypectomy may be performed as an open procedure. Conventional polypectomys are completed using various apparatus and techniques known in the art.

As noted above, there are two forms of polyps, sessile and pedunculated. The stalkless or sessile polyps are generally removed using electrical forceps. The excess tissue projecting from the mucous membrane is cauterized, sealed, or the like, and torn from the tissue wall. Large sessile polyps or pedunculated polyps (e.g., polyps having stalks) tend to be relatively larger with a greater blood supply. The size and shape of large sessile polyps or pedunculated polyps typically do not lend themselves to being removed using traditional forceps. Unlike sessile polyps, large sessile polys or pedunculated polyps cannot simply be grasped in the jaw members of an electric forceps and torn from the tissue wall. Instead, the polypectomy is performed using a surgical snare device. The snare device is configured with a snare for looping over the hanging polyp and fitting securely over the polyp and/or poly stalk. By constricting the snare, and selectively applying energy, the device may cauterize or seal the polyp along the stalk as the polyp is severed from the tissue wall.

Polyp removal using a surgical snare device requires an operator to loop the snare over the end of the polyp in order to properly position the polyp about the stalk. In many circumstances access to the stalk of the polyp, much less the entire polyp is limited. Without complete access to the polyp the surgical snare device is useless for removal of the polyp.

It would therefore be beneficial to have a polyp removal device that does not incorporate a snare that must be placed looped over a polyp.

Reference is made to EP 0 795 301 A1, which discloses a bipolar cutting and coagulation instrument comprising an end effector including a triangular first electrode adapted to pivot between an open and closed position and second and third electrodes arranged substantially parallel to the first electrode when in the closed position. The second and third electrodes may be joined by a central region including a channel adapted to receive a cutting edge of the first electrode.

### SUMMARY

The invention is defined by the independent claim below. Dependent claims are directed to optional features and preferred embodiments.

The present disclosure relates to apparatus and methods for the removal of polyps and the like.

An apparatus for the removal of tissue is disclosed which comprises a first jaw member including first and second elongated members, the first and second elongated members having proximal and distal ends and defining a channel therebetween; and a second jaw member having proximal and distal ends and defining a tissue contacting surface, the proximal end of the second jaw member being pivotably coupled between the proximal ends of the first and second elongated members, wherein the distal end of the second jaw member is in a spaced apart relationship with the first jaw member when in a first position, and the distal end of the second jaw member extends into the channel defined between the distal ends of first and second elongated members when in a second position.

The distal end of the second jaw member may be configured to extend beyond the distal end of first and second elongated members of the first jaw member when the jaws are in the second position. The second jaw member may be configured to deliver electrosurgical energy to the tissue.

The first and second elongated members include a shelf extending at least partially therebetween. An electrode may be supported on the shelf. The shelf may be configured to extend distally between the first and second elongated members to form a stop for engaging a distal end of the second jaw member when the jaws are in the second position. An electrode may be operably disposed between the first and second elongated members.

The first and second jaw members may be resilient. At least one of the first jaw member and the second jaw member includes a semi-arcuate shape. Proximal ends of the first and second jaw members may be configured to be operably engaged with an endoscopic device.

The first and second jaw members may be configured for bipolar sealing of tissue. The first jaw may be at least partially electrically conductive, and wherein the second jaw may be electrically non-conductive and includes at least one electrode disposed between the first and second elongated members thereof.

An apparatus for the removal of tissue is disclosed and includes a first jaw member having first and second elongated space apart members and a shelf disposed therebetween; and a second jaw member pivotably connected between the first and second elongated members of the first jaw member, wherein the second jaw member is configured to operably engage the shelf when the first and second jaw members are in a closed position.

A gap is formed between the first and second jaw members when the first and second jaw members are in a closed position. The first and second jaw members may be configured for a bipolar electrosurgical procedure. At least one of the first jaw member and the second jaw member may be configured for a monopolar electrosurgical procedure.

The first and second jaw members may be formed from a resilient material.

The apparatus may further include a housing for receiving the first and second jaw members therein. The first and second jaw members may be flexed for receipt within the housing.

A system for the removal of tissue is disclosed. The system comprises an apparatus having first and second jaw members configured to selectively receive tissue therebetween. The first jaw member includes first and second elongated members, the first and second elongated members having proximal and distal ends and defining a channel therebetween; and the second jaw member includes proximal and distal ends and defines a tissue contacting surface, the proximal end of the second jaw member being pivotably coupled between the proximal ends of the first and second elongated members, wherein the distal end of the second jaw member is in a spaced apart relationship with the first jaw member when in a first position, and the distal end of the second jaw member extends into the channel defined between the distal ends of first and second elongated members when in a second position. The system further includes a source of electrosurgical energy operably connected to at least one of the jaw members to deliver electrosurgical energy to the tissue.

### BRIER DESCRIPTION OF THE DRAWINGS

The foregoing summary, as well as the following detailed description will be better understood when read in conjunction with the appended figures. For the purpose of illustrating the present disclosure, a preferred embodiment is shown. It is understood, that the present disclosure is not limited to the precise arrangement and instrumentalities shown.
FIG. 1 is a perspective view of polyp removal jaws according to an embodiment of the present disclosure, in a first or open position;
FIG. 2 is a perspective view of the polyp removal jaws of FIG. 1, shown in a second or closed position;
FIG. 3 is partial, cross-sectional, side view of the polyp removal jaws of FIGS. 1 and 2, shown in a closed position, and shown operably connected to an endoscopic device;
FIG. 4 is a partial, cross-sectional, side view of the polyp removal jaws of FIGS. 1-3, shown in an open position, operably connected to an endoscopic device;
FIG. 5A is a cross-sectional, side view of the polyp removal jaws of FIGS. 1-4, shown in a closed position;
FIGS. 5B-5F are transverse, cross-sectional views of the polyp removal jaw of FIGS. 1-5A taken along lines 5B-5B, 5C-C, 5D-5D, 5E-5E and 5F-5F, respectively, of FIG. 5A;
FIG. 6 is a perspective view of polyp removal jaws according to an alternate embodiment of the present disclosure, shown in a first or open position;
FIG. 7 is a perspective view of the polyp removal jaws of FIG. 6, shown in a second or closed position;
FIG. 8 is a perspective view of polyp removal jaws according to another embodiment of the present disclosure, shown in first or open position;
FIG. 9 is a perspective view of the polyp removal jaws of FIG. 8, shown in a second or closed position;
FIG. 10A is a cross-sectional, side view of the polyp removal jaws of FIGS. 8-9, shown in a closed position;
FIGS. 10B-10E are transverse, cross-sectional views of the polyp removal jaws of FIGS 8-10A taken along line 10B-10B, 10C-10C, 10D-10D and 10E-10E, respectively, of FIG. 10A;
FIGS. 11A-11D are partial, cross-sectional, side views of polyp removal jaws shown at various stages of insertion into a tubular housing; and
FIG. 12 is a schematic illustration of an electrosurgical system including any of the jaws shown in FIGS. 1-11D.

### DETAILED DESCRIPTION OF EMBODIMENT

The foregoing summary, as well as the following detailed description will be better understood when read in conjunction with the appended figures. For the purpose of illustrating the present disclosure, various embodiments are shown. It is understood, however, that the present disclosure is not limited to the precise arrangement and instrumentalities shown. As shown in the drawings and described throughout the following description, as is traditional when referring to relative positioning on an object, the term "proximal" refers to the end of the apparatus which is closer to the user and the term "distal" refers to the end of the apparatus which is further from the user.

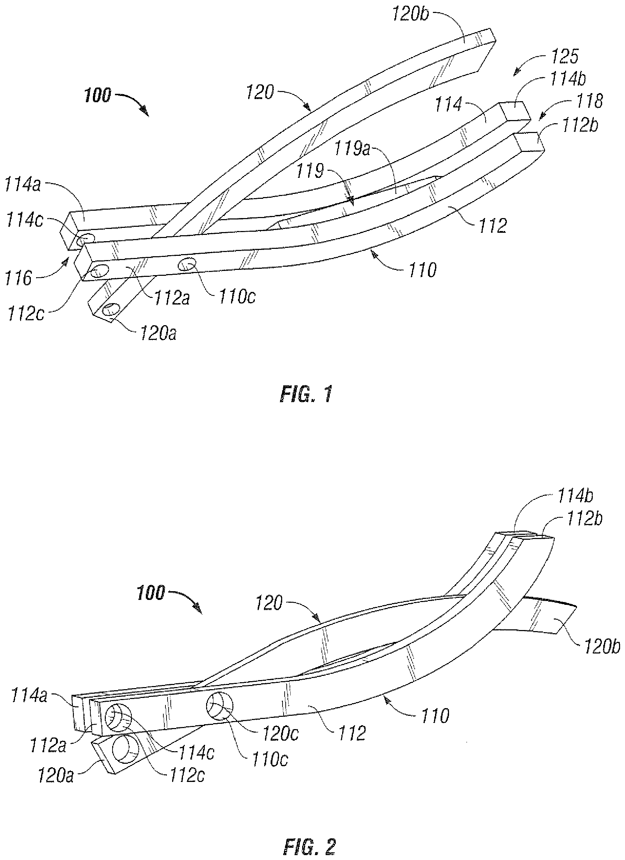

Referring to FIGS. 1-5F, an illustrative embodiment of the presently disclosed polyp removal jaws is shown therein and generally designated as jaws 100. Jaws 100 comprise a first jaw member 110 and a second jaw member 120. First and second jaw members 110, 120 are pivotably connected to one another by a pivot pin 115. Jaw members 110, 120 are configured to retain, seal, cauterize, and/or sever tissue grasped therebetween. Jaw members 110, 120 are configured to engage tissue to be excised, such as stems of pedunculated polyps.

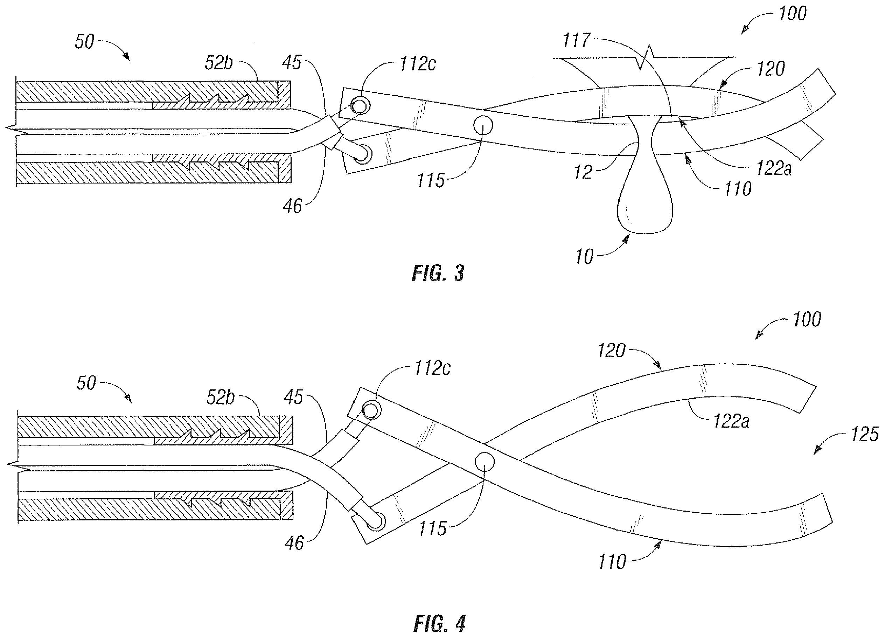

Jaws 100 may be incorporated into a hand-held instrument for use in open surgical procedures or may be configured, as shown in FIGS. 3 and 4, for incorporation into endoscopic instruments for use in closed surgical procedures. First and second jaw members 110, 120 may be composed of plastics, polymers, mentals, alloys or the like or any combination thereof. First and second jaw members 110, 120 may be fabricated from any suitable dielectric material. Jaws 100 may be configured for monopolar and/or bipolar operation, as will be discussed below.

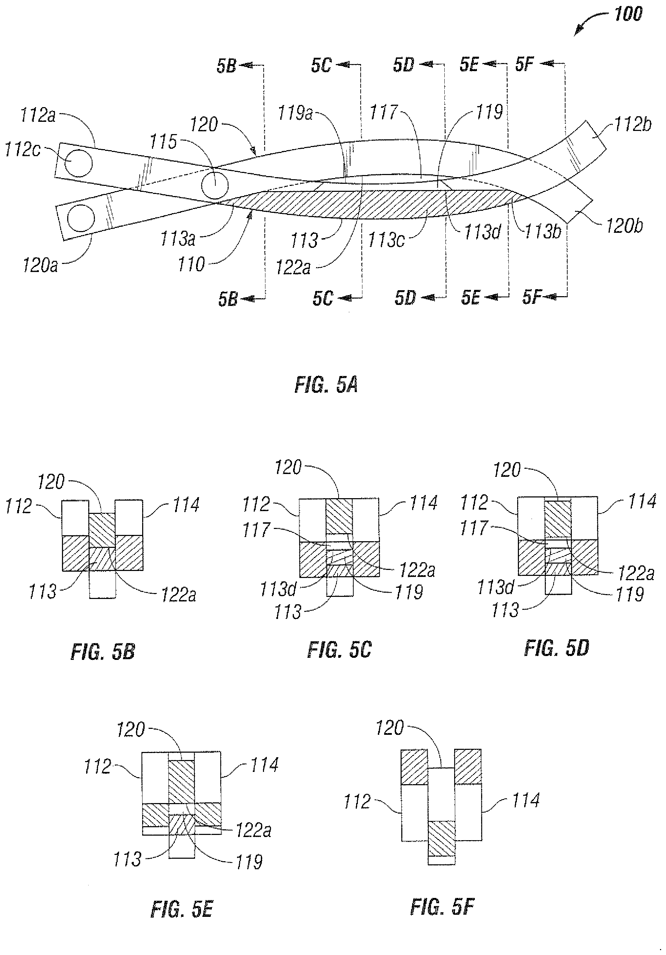

First jaw member 110 forms a semi-arcuate base including a first elongated section 112 and a second elongated section 114 spaced apart from substantially parallel to first elongate section 112. A connector or shelf member 113 (FIGS. 5A-5E) extends at least partially between first and second elongated sections 112, 114 to one another. First and second elongated sections 112, 114 include proximal ends 112a, 114a and distal ends 112b, 114b, respectively.

Proximal ends 112a, 114a of first and second elongated sections 112, 114 define a first channel 116 therebetween. First channel 116 is configured for selectively receiving a proximal end 120a of second jaw member 120 therein, as will be described in greater detail below. Distal ends 112b, 114b of elongated sections 112, 114, define a second channel 118 therebetween. Second channel 118 is configured for selectively receiving a distal end 120b of second jaw member 120 therethrough. Proximal ends 112a, 114a of first and second elongated sections 112, 114, respectively, define an opening 110c for receiving a pivot pin 115.

With continued reference to FIGS. 1-5F, second jaw member 120 forms a single semi-arcuate member having proximal and distal ends 120a, 120b, respectively. Proximal end 120a of second jaw member 120 is configured to be received within first channel 116 formed between proximal ends 112a, 114a of respective first and second elongated sections 112, 114, respectively. Proximal end 120a of second jaw member 120 defines opening 120c corresponding to openings 110c formed in first and second elongated section 112, 114. Opening 120c is configured for receiving pivot pin 115. Distal end 120 of second jaw member 120 is configured to be selectively received within and through second channel 118 formed between distal ends 112b, 114b of respective first and second elongated sections 112, 114.

Second jaw member 120 may comprise an electrically conductive material or may be fabricated substantially of an electrically conductive material. In an alternate embodiment, second jaw member 120 may include one or more electrodes (not shown), supported thereon, for effecting monopolar and/or bipolar cutting and/or sealing of tissue. Second jaw member 120 may include an insulative layer (not shown) to prevent a short circuit with first jaw member 110 and/or the one or more electrodes mounted thereon. The insulative layer may also prevent damage to tissue resulting from incidental contact during a surgical procedure.

Referring now to FIGS. 3-4 and 5A-5F, second jaw member 120 includes a tissue contacting surface 122a. Tissue contacting surface 122a may comprise at least a portion of a length of second jaw member 120. As will be discussed in more detail below, as first and second jaw members 110, 120 are closed about stem 12 of polyp 10 (FIG. 3), tissue contacting surface 122a of second jaw member 120 engages and guides stem 12 against first and second jaw members 110, 120. Tissue contacting surface 122a may have a rounded, sharpened, flattened or other suitable transverse cross-sectional profile.

Proximal ends 112a, 114a of first and second elongated sections 112, 114, respectively, and proximal end 120a of second jaw member 120, may further be configured for incorporation into a conventional hand-held forceps or for operable engagement with the distal end of an endoscopic device. With particular reference to FIGS. 3 and 4, proximal ends 112a, 114a of first and second elongated sections 112, 114 define openings 112c, 114c (FIGS. 1 and 2) therein, configured for operable engagement with actuation cables 45, 46 extending from a distal end 52b of an endoscopic device 50. As will be described in more detail below, first and second actuation cables 45, 46 are configured to move first and second jaw members 110, 120. First and second actuation cables 45, 46 may operate in unison or independently of each other to pivot first and/or second jaw members 110, 120 relative to one another. Actuation cables 45, 46 may also be configured to supply electrosurgical energy to first and/or second jaw members 110, 120, respectively, and/or alternatively, to electrodes (not shown) mounted thereon.

Referring now, to FIGS. 5A-5F, shelf member 113 is disposed between first and second elongated section 112, 114. Shelf member 113 includes a proximal end 113a, a distal end 113b and a middle portion 113c therebetween. Proximal and distal ends 113a, 113b are configured to act as stops when first and second jaw members 110, 120 are in a closed and fully engaged position with one another. Proximal end 113a of shelf member 113 is configured to engage second jaw member 120 in a region proximal of tissue contacting surface 122a (FIGS. 5A and 5B). Distal end 113b of shelf member 113 is configured to engage second jaw member 120 in a region distal of tissue contacting surface 122a (FIGS. 5A and 5E). In this manner, a gap or opening 1 17 is formed between tissue contacting surface 122a of second jaw member 120 and an upper surface 113d of shelf member 113 of first jaw member 110 (FIGS. 5C and 5D). By varying the curvature of first and/or second jaw members 110, 120 and/or altering the configuration of proximal and/or distal ends 113a, 113b of shelf member 113, the height or dimensions of opening 117 may be adjusted. Proximal and distal ends 113a, 113b of shelf member 113 may include a layer of insulation to prevent a short circuit between first and second jaw members 110, 120.

Middle portion 113c of shelf member 113 may be recessed with, flush to or extend past an upper surface of first and second elongated sections 112, 114. Middle portion 113c may be flat, curved inwardly, curved outwardly, or may include a texture for more securely engaging tissue. Depending on the electrical configuration of jaw 100, middle portion 113c of shelf member 113 may include one or more electrodes 119 mounted thereon. Electrode 119 may be sized and dimensioned to be maintained on middle portion 113c of shelf member 113. Electrode 119 may be recessed within channels 116, 118 formed between first and second elongated sections 112, 114. Alternately, electrode 119 may be maintained flush with the top surface of elongated sections 112, 114 or may extend beyond the top surface of elongated sections 112, 114. Electrode 119 may have a flat, curved or textured tissue contacting surface 119a.

Referring back to FIG. 1 and 4, polyp removal jaw 100 is shown in a first or open position. In the open position, distal end 120b of second jaw member 120 is pivoted out from within channel 118 formed between distal ends 112b, 114b of first and second elongated sections 112, 114 to define an opening 125 between first and second jaw members 110, 120. Opening 125 is configured for facilitating the placement of jaws 100 about a portion of tissue, such as, for example the stem of a pedunculated polyp. Depending on the configuration of the actuation mechanism, and whether it is for open or closed procedures, first jaw member 110 may be held stationary relative to the actuation assembly (not shown) while second jaw member 120 is pivoted about pivot pin 115 relative to first jaw member 110. In an alternate embodiment, both first and second jaw members may be pivoted relative to each, while in yet another embodiment, second jaw member 120 may be held stationary relative to the actuation assembly while first jaw member 110 is pivoted about pivot pin 115 relative to second jaw member 120. First and second jaw member 110, 120 may be articulated up to and beyond ninety degrees (90°) relative to one another. The range of articulation of second jaw member 120 relative to first jaw member is limited only by range of motion of the actuation assembly connected thereto.

Turning now to FIGS. 2-3 and 5A-F, polyp removal jaw 100 is shown in a second or closed position. In the closed position, proximal and distal ends 110a, 110b of second jaw member 110 extend through or positioned in channels 116, 118, respectively, formed between elongated sections 112, 114. As described above, shelf member 113 includes proximal and distal ends 113a, 113b for engaging second jaw member 120. Gap 119 formed between first and second jaw members 110, 120 is configured for operably retaining stem 12 of a polyp 10 (FIG. 3). Whether of a monopolar or bipolar design, electrosurgical energy may be applied to either first or second jaw members 110, 120 or one or more electrodes 119 mounted thereon, at any time during the polyp removal procedure. For larger polyps it may be necessary to activate the tissue sealing mechanism prior to the complete closure of first and second jaw members 110, 120.

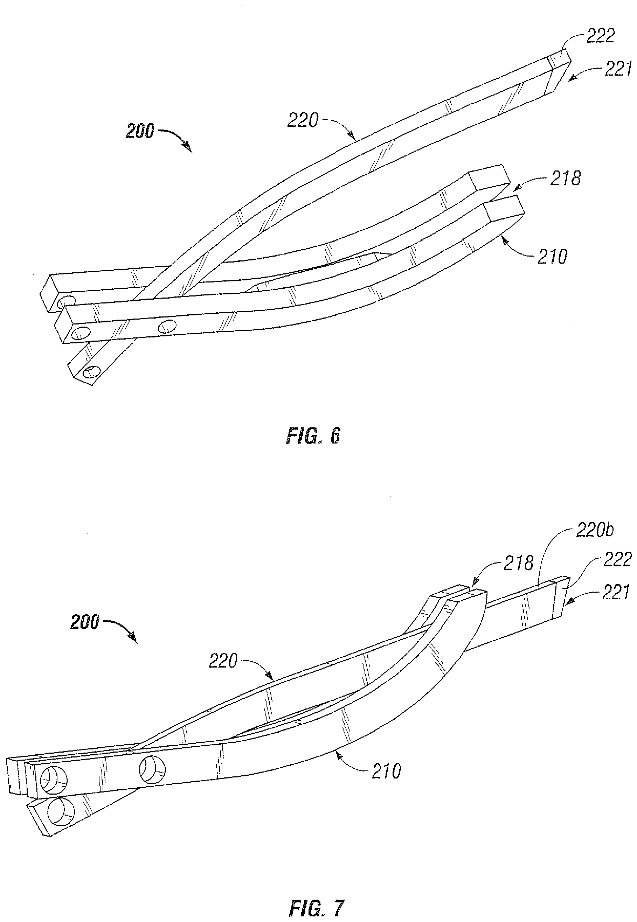

Referring now to FIGS. 6 and 7, polyp removal jaws according to an alternate embodiment of the present disclosure are shown as 200. Polyp removal jaws 200 are substantially similar to polyp removal jaws 100 and will only be described to the extent necessary to disclose the difference in construction and operation between the two. Polyp removal jaws 200 include first and second jaw members 210, 220. Second jaw member 220 includes a distal end 220b configured for supplying electrosurgical energy by way of an electrode tip 222. When jaws 200 are in a closed position, as shown in FIG. 7, distal end 220b of second jaw member 220 extends through channel 218 of first jaw member 210 and defines an operational end 221 that acts as a monopolar pencil. Operational end 221 may be an extension of electrically conductive second jaw member 220. Alternatively, operational end 221 may include an electrode or electrode tip 222. Electrode tip 222 may be selectively energized to permit a user to spot cauterize tissue without having to introduce a second instrument into the surgical field.

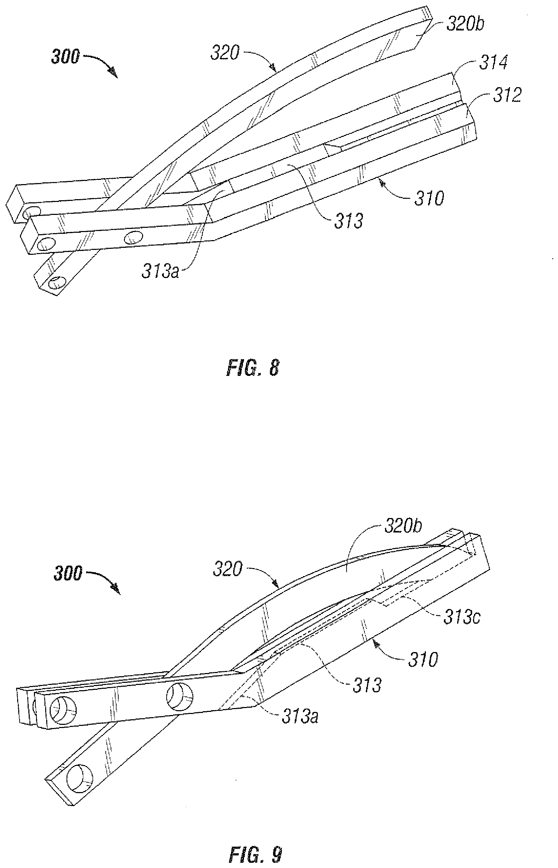

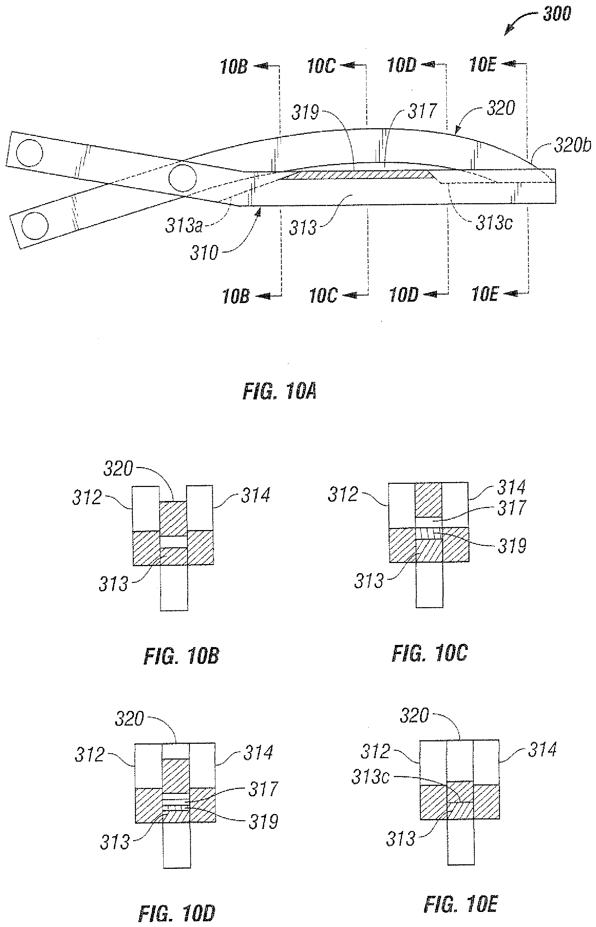

Referring now to FIGS. 8-10E, a second embodiment of the polyp removal jaw 300 is illustrated. Polyp removal jaws 300 are substantially similar to polyp removal jaws 100, 200 and will only be described with respect to the difference in construction and operation therebetween. Polyp removal jaw 300 includes a first and second jaw member 310, 320. First jaw member 310 forms a level, or planar, or linear base including a first and second elongated section 312, 314 and a shelf 313 therebetween. In the present embodiment, shelf 313 extends distally between first and second elongated members 312, 314, substantially along an entire length of elongated members 312, 314, thereby preventing distal end 320b of second jaw member 320 from extending through elongated members 312, 314.

Turning now to FIGS. 10A-10E in particular, shelf member 313 is disposed between first and second elongated section 312, 314. Shelf member 313 extends distally the length of first and second elongated section 312, 314. Shelf member 313 defines a closure surface 313c configured to engage a distal end 320b of second jaw member 320 (FIGS. 10A and 10E) when jaws 300 are in a closed position. Proximal end 313a of shelf member 313 may be configured to engage a portion of second jaw member 320, or as shown (FIGS. 10A and 10B), proximal end 313a does not engage any portion of second jaw member 320. Shelf member 313 may include an electrode 319 mounted thereon (FIGS. 10A and 10C-D). Electrode 319 may be configured similar to electrode 119, as detailed above. A gap 317 is formed between first and second jaw members 310, 320. The height or dimension of gap 317 may vary depending on the configuration, i.e. arc, length, of first and second jaw members 310, 320.

In an alternate embodiment, closure surface 313c of shelf 313 may be configured to securely engage distal end 320b of second jaw member 320. Alternatively, closure surface 313c may be configured to complete a circuit upon contact with distal end 320b of second jaw member 320. In yet another embodiment, second jaw member 320 may be configured to deform as distal end 320b engages closure surface 313c of shelf 313. In this manner, the height of gap 317 between first and second jaw member 310, 320 will be reduced as first and second jaw members 310, 320 are squeezed together. As with prior embodiments, polyp removal jaw 300 may be monopolar, bipolar or a combination of the two.

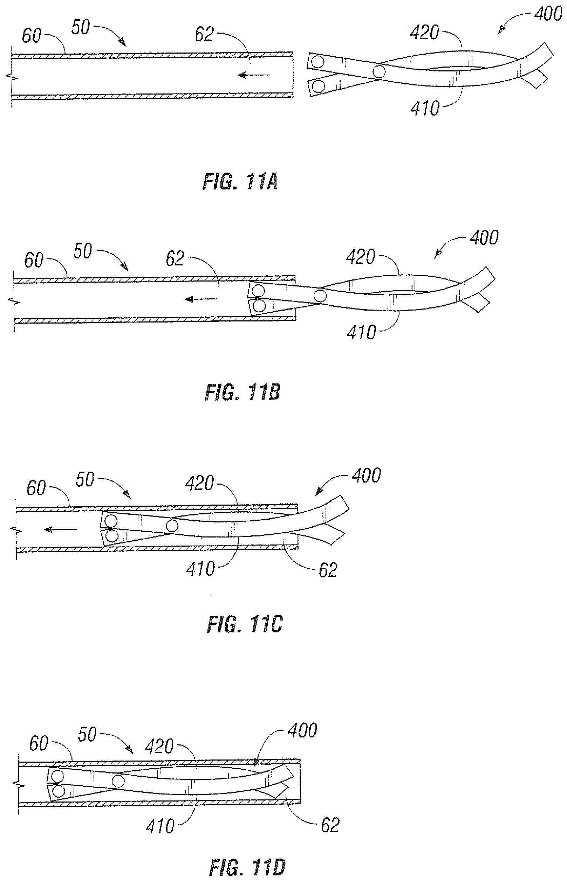

Referring now to FIGS. 11A-11D, polyp removal jaws 100, 200, 300 herein described may be configured to be received with a housing 60 of an endoscopic device 50, even when on opening 62 in housing 60 is less then the height of the jaws in a first or closed state. Polyp removal jaws 400 may be configured from elastomeric material, shape memory metals, plastics or the like. Referring initially to FIG. 11A, polyp removal jaws 400 include first and second jaw member 410, 420. When jaws 400 are exposed or deployed from housing 60, the height of polyp removal jaw 400 is greater than the dimension of opening 62 of housing 60 from which it is to be received. A polyp removal jaw 400 is retracted into housing 50, first and second jaw members 410, 420 are cammed against a front edge of housing 60 so as to be extended and compressed towards one another (10B-11C) until polyp removal jaws 400 are dimensioned to be completely received within housing 60. Polyp removal jaws 400 are configured to return to their initial shape upon ejection or deployment from housing 60. Polyp removal jaws 400 may be configured in any manner herein described.

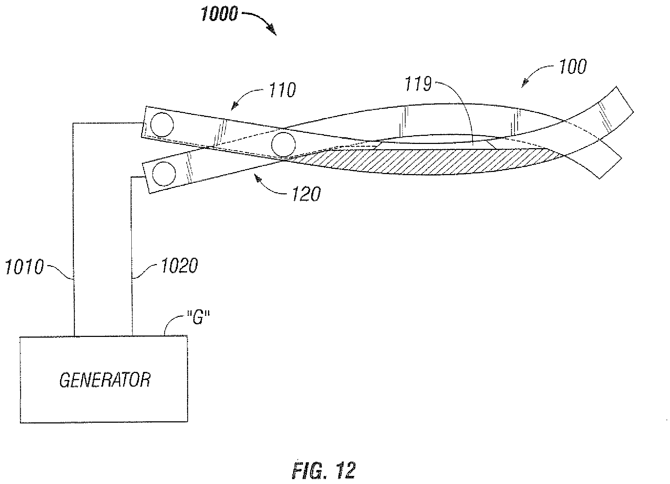

As seen in FIG. 12, any of the polyp removal jaws disclosed herein, such as, for example, polyp removal jaws 100 (as exemplarily shown in FIG. 12) may form a part of an electrosurgical system 1000. Electrosurgical system 100 may include at least an electrosurgical generator "G", and polyp removal jaws 100 electrically connected/connectable to electrosurgical generator "G" or the like via electrical conduits 1010, 1020. In particular, as seen in FIG. 12, a first electrical conduit 1010 is electrically connected to electrode 119 of first jaw 110 of polyp removal jaws 100, and a second electrical conduit 1020 is electrically connected to second jaw 120 of polyp removal jaws 100.

Thus, it should be understood that various changes in form, detail and operation of the polyp removal jaws of the present disclosure may be made without departing from the scope of the present invention as defined by the appended claims.

## Claims

1. An apparatus (100; 200; 300; 400) for the removal of tissue comprising:
a first jaw member (110) including first (112) and second (114) elongated spaced apart members and a shelf (113) disposed therebetween; and
a second semi-arcuate jaw member (120) pivotably connected between the first and second elongated members of the first jaw member, the distal end of the second jaw member being in a spaced apart relationship with the first jaw member in an open position and extending into a channel defined by the distal ends of the first and second elongated members in a closed position, wherein:
in the closed position a gap (117) is formed between the first and second jaw members suitable for retaining the stem (12) of a polyp (10); and
the second jaw member is configured to operably engage the shelf when the first and second jaw members are in a closed position.

2. The apparatus of claim 1, wherein the first and second jaw members are configured for a bipolar electrosurgical procedure.

3. The apparatus of claim 1, wherein at least one of the first jaw member and the second jaw member is configured for a monopolar electrosurgical procedure.

4. The apparatus of claim 1, wherein the first and second jaw members are formed from a resilient material.

5. The apparatus of claim 5, further comprising a housing (60) for receiving the first and second jaw members therein, wherein the first and second jaw members are flexed for receipt within the housing.

6. Apparatus as claimed in any one of the preceding claims, with an electrode (119) supported on the shelf.

7. Apparatus as claimed in any one of the preceding claims, wherein the first jaw member is semi-arcuate.

8. Apparatus as claimed in any one of the preceding claims, wherein the first and second jaws are pivotably coupled to each other at their proximal ends (112a, 114a, 120a).

9. Apparatus as claimed in any one of the preceding claims, in which the shelf has a proximal portion (113a), a middle portion (113c), and a distal portion (113b) with the proximal and distal portions serving as a stop.

10. Apparatus as claimed in claim 8, wherein the middle portion is recessed with respect to an upper surface of the spaced apart members of the first jaw member.

11. Apparatus as claimed in claim 8 or 9, in which the middle portion is textured to engage tissue.

12. Apparatus as claimed in claim 8, 9 or 10, in which the tissue-contacting surface of the middle portion is curved.

13. Apparatus as claimed in any one of the preceding claims, wherein the second jaw member passes between the first and second spaced-apart elongate members of the first jaw when moving to the closed position, until the distal end of the second jaw has advanced beyond the space between the elongate members.

## Patentansprüche

1. Vorrichtung (100; 200; 300; 400) zum Entfernen von Gewebe, umfassend:
ein erstes Klemmbackenelement (110), das erste (112) und zweite (114) langgestreckte Elemente aufweist, die voneinander beabstandet sind, und eine Ablage (113), die dazwischen angeordnet ist; und
ein zweites, halbbogenförmiges Klemmbackenelement (120), das schwenkbar zwischen den ersten und zweiten langgestreckten Elementen des ersten Klemmbackenelements verbunden ist, wobei das distale Ende des zweiten Klemmbackenelements in einer beabstandeten Beziehung mit dem ersten Klemmbackenelement ist, wenn es in einer offenen Position ist, und sich in einen Kanal erstreckt, der zwischen den distalen Enden der ersten und zweiten langgestreckten Elemente definiert ist, wenn es in einer geschlossenen Position ist, wobei:
in der geschlossenen Position ein Spalt (117) zwischen den ersten und zweiten Klemmbackenelementen gebildet wird, der geeignet ist, um den Stiel (12) eines Polypen (10) zu halten; und
das zweite Klemmbackenelement konfiguriert ist, um funktionsmäßig mit der Ablage in Eingriff zu treten, wenn die ersten und zweiten Klemmbackenelemente in einer geschlossenen Position sind.

2. Vorrichtung nach Anspruch 1, wobei die ersten und die zweiten Klemmbackenelemente für ein bipolares elektrochirurgisches Verfahren konfiguriert sind.

3. Vorrichtung nach Anspruch 1, wobei mindestens eines von den ersten und zweiten Klemmbackenelementen für ein monopolares elektrochirurgisches Verfahren konfiguriert ist.

4. Vorrichtung nach Anspruch 1, wobei die ersten und zweiten Klemmbackenelemente aus einem elastischen Material hergestellt wurden.

5. Vorrichtung nach Anspruch 5, ferner umfassend ein Gehäuse (60) für das Aufnehmen der ersten und zweiten Klemmbackenelemente darin, wobei die ersten und zweiten Klemmbackenelemente zur Aufnahme innerhalb des Gehäuses gebogen sind.

6. Vorrichtung nach einem der vorhergehenden Ansprüche, mit einer Elektrode (119), die auf der Ablage gelagert ist.

7. Vorrichtung nach einem der vorhergehenden Ansprüche, wobei das erste Klemmbackenelement halbbogenförmig ist.

8. Vorrichtung nach einem der vorhergehenden Ansprüche, wobei die ersten und zweiten Klemmbackenelemente schwenkbar an ihren proximalen Enden (112a, 114a, 120a) miteinander gekoppelt sind.

9. Vorrichtung nach einem der vorhergehenden Ansprüche, wobei die Ablage einen proximalen Abschnitt (113a), einen mittleren Abschnitt (113c) und einen distalen Abschnitt (113b) aufweist, wobei der proximale und der distale Abschnitt als ein Anschlag dienen.

10. Vorrichtung nach Anspruch 8, wobei der mittlere Abschnitt in Bezug auf eine obere Fläche der beabstandeten Elemente des ersten Klemmbackenelements zurückgesetzt ist.

11. Vorrichtung nach Anspruch 8 oder 9, wobei der mittlere Abschnitt strukturiert ist, um in Gewebe einzugreifen.

12. Vorrichtung nach Anspruch 8, 9 oder 10, wobei die Fläche des mittleren Abschnitts, die mit dem Gewebe in Kontakt kommt, gekrümmt ist.

13. Vorrichtung nach einem der vorangegangenen Ansprüche, wobei das zweite Klemmbackenelement zwischen den ersten und zweiten beabstandeten langgestreckten Elementen der ersten Klemmbacke verfährt, wenn es sich in eine geschlossene Position bewegt, bis das distale Ende der zweiten Klemmbacke über den Raum zwischen den langgestreckten Elementen hinaus verfahren ist.

## Revendications

1. Appareil (100 ; 200 ; 300 ; 400) pour l'enlèvement de tissu comprenant :
un premier élément de mâchoire (110) incluant des premier (112) et second (114) éléments allongés espacés et un rebord (113) disposé entre ces derniers ; et
un second élément formant mâchoire semi-curviligne (120) relié de manière pivotante entre les premier et second éléments allongés du premier élément formant mâchoire, l'extrémité distale du second élément formant mâchoire étant dans une relation espacée avec le premier élément formant mâchoire dans une position ouverte et s'étendant dans un canal défini par les extrémités distales des premier et second éléments allongés dans une position fermée, dans lequel :
dans la position fermée un espacement (117) est formé entre les premier et second éléments formant mâchoires, approprié pour retenir la racine (12) d'un polype (10) ; et
le second élément formant mâchoire est configuré pour mettre en prise de manière opérationnelle le rebord lorsque les premier et second éléments formant mâchoires sont dans une position fermée.

2. Appareil selon la revendication 1, dans lequel les premier et second éléments formant mâchoires sont configurés pour une procédure électrochirurgicale bipolaire.

3. Appareil selon la revendication 1, dans lequel au moins un du premier élément formant mâchoire et du second élément formant mâchoire est configuré pour une procédure électrochirurgicale monopolaire.

4. Appareil selon la revendication 1, dans lequel les premier et second éléments formant mâchoires sont formés d'une matière élastique.

5. Appareil selon la revendication 5, comprenant en outre un logement (60) pour recevoir les premier et second éléments formant mâchoires en son sein, dans lequel les premier et second éléments formant mâchoires sont fléchis pour réception à l'intérieur du logement.

6. Appareil selon l'une quelconque des revendications précédentes, ayant une électrode (119) supportée sur le rebord.

7. Appareil selon l'une quelconque des revendications précédentes, dans lequel le premier élément formant mâchoire est semi-curviligne.

8. Appareil selon l'une quelconque des revendications précédentes, dans lequel les première et seconde mâchoires sont accouplées de manière pivotante l'une à l'autre au niveau de leurs extrémités proximales (112a, 114a, 120a).

9. Appareil selon l'une quelconque des revendications précédentes, dans lequel le rebord a une portion proximale (113a), une portion médiane (113c) et une portion distale (113b), les portions proximale et distale servant de butée.

10. Appareil selon la revendication 8, dans lequel la portion médiane est en retrait par rapport à une surface supérieure des éléments espacés du premier élément formant mâchoire.

11. Appareil selon la revendication 8 ou 9, dans lequel la portion médiane est crantée pour mettre en prise du tissu.

12. Appareil selon la revendication 8, 9 ou 10, dans lequel la surface de la portion médiane entrant en contact avec du tissu est courbée.

13. Appareil selon l'une quelconque des revendications précédentes, dans lequel le second élément formant mâchoire passe entre les premier et second éléments allongés espacés de la première mâchoire en se déplaçant vers la position fermée, jusqu'à ce que l'extrémité distale de la seconde mâchoire se soit avancée au-delà de l'espace entre les éléments allongés.
